# EUROPEAN PATENT SPECIFICATION

(11) **EP 0 395 046 B1**
(45) Date of publication and mention of the grant of the patent: **29.12.1993**
(21) Application number: 90107920.2
(22) Date of filing: 26.04.1990
(51) Int. Cl.: C07C 249/04, C07C 251/44

(54) **Process for oxidizing saturated primary amines to oximes**
Verfahren zur Oxidation von gesättigten primären Aminen zu Oximen
Procédé d'oxidation d'amines saturés en oximes

(30) Priority: 27.04.1989 IT 2029389
(43) Date of publication of application: 31.10.1990
(73) Proprietor: ENICHEM S.p.A., 20124 Milano (IT)
(72) Inventor: Venturello, Carlo, I-28100 Novara (IT); D'Aloisio, Rino, I-28100 Novara (IT)
(74) Representative: Weinhold, Peter, Dr.

(56) References cited:
- EP-A- 0 043 445
- EP-A- 0 267 362
- DE-C- 1 021 358
- US-A- 4 504 681

## Description

The present invention relates to a process for preparing aliphatic and cycloaliphatic oximes by reacting, in the liquid phase, the corresponding saturated primary aliphatic or cycloaliphatic amine with oxygen in the presence of catalysts based on compounds of metals of Group IV B of the Periodic System, i.e. titanium, zirconium and hafnium.

The (cyclo)aliphatic oximes obtainable thereby are products which have useful applications as such, for example as oxidation inhibitors, or represent interesting intermediates for the production of amides with corresponding wide applicative possibilities.

In particular, in case the amine is cyclohexylamine, the resulting cyclohexanone oxime can be converted further to the corresponding cyclic amide, i.e., epsilon-caprolactam.

Said caprolactam is the starting monomer for the production of nylon 6.

Already known is the oxidation to oximes of saturated aliphatic or cycloaliphatic primary amines (e.g. cyclohexylamine) which have a C-H linkage in alpha position with respect to the amine group with hydrogen peroxide in the presence of catalysts based on molybdenum, tungsten and uranium, or with organic hydroperoxides in the presence of catalysts based on titanium, molybdenum, tungsten and vanadium in the organic phase.

Nevertheless, the use of the above oxidizing agents is expensive and, in the case of the utilization of an organic hydroperoxide, further complications associated with the separation, purification and the like can occur due to the presence, in the final reaction mixture, of by-products (alcohols) deriving from the reduction of the hydroperoxide.

In addition to these drawbacks, there are the known operative risks in handling peroxide compounds.

On the other hand it is also known (cf. US-A-4,504,681) to prepare oximes from saturated (cyclo)aliphatic primary amines of the above type by using oxygen as oxidant and working in the gas phase and in the presence of solid catalysts based on SiO₂ gel and gamma-A1₂O₃, optionally associated with tungsten oxides.

Said process involves relatively severe working conditions due to the gas phase (temperatures approximately ranging from 120°C to 250°C, etc.).

On the other hand it was assumed so far that metals such as titanium generally are only efficient activators for peroxide compounds.

In contrast thereto, it has now surprisingly been found that the oxidation reaction of saturated (cyclo)aliphatic primary amines to oximes can be carried out by using oxygen in the liquid phase and employing catalysts based on compounds of metals belonging to Group IV B of the Periodic System.

Thus, according to the present invention, aliphatic and cycloaliphatic oximes, in which the (cyclo)aliphatic residue may optionally be substituted by one or more groups which are inert under the reaction conditions and preferably contains from 2 to 15 carbon atoms, are prepared by reaction of the corresponding saturated (cyclo)aliphatic primary amine, which contains a C-H linkage in alpha position with respect to the amine group, with oxygen, said reaction being conducted in the liquid phase and in the presence of a catalyst comprising at least one compound of a metal of Group IV B of the Periodic System.

The saturated (cyclo)aliphatic amine, which is the starting substrate, preferably has the formula: 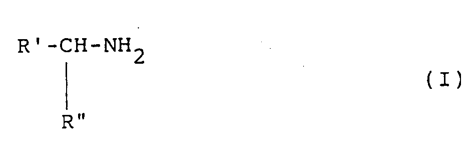
in which R' represents a straight or branched alkyl group, R'' represents hydrogen or a straight or branched alkyl group, the same or different from R', or R' and R'', taken together, give rise to a cycloalkylene ring, the total of carbon atoms contained in R' and R'' being up to 15 and R' and R'' being optionally substituted by one or more groups inert under the reaction conditions.

The oxidation reaction may schematically be represented as follows:

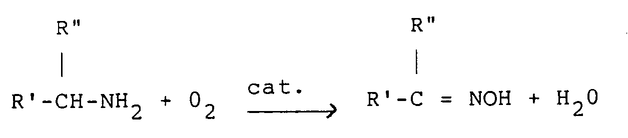

The process of the present invention is conducted in the liquid phase which comprises the saturated primary amine substrate which is utilized as such or in the form of a solution thereof, in the presence of a catalyst based on compounds of metals of Group IV B of the Periodic System (Ti, Zr, Hf).

The starting amine substrate may be substituted by groups inert under the oxidation conditions, for example, lower (C₁₋₄-)alkyl groups, (C₁₋₄-)alkoxy groups and halogen atoms (F, Cl, Br and I), etc. Besides cyclohexylamine, also cyclopentylamine, (cyclo)heptylamine, cyclododecylamine etc. have proven to be particularly advantageous substrates.

The catalysts used according the present invention may generally be defined as compounds based on Ti, Zr and/or Hf (Group IV B of the Periodic System) in the form of salts, oxides, the latter optionally associated with Si0₂, or of metallorganic compounds, said compounds being at least partially soluble in the organic reaction medium consisting of the amine substrate as such or, alternatively, of the substrate dissolved in a suitable solvent.

When a metallorganic compound is utilized, the process of the present invention preferably is conducted in the presence of at least one compound selected from the metallorganic compounds of formula (II):

Rₘ (0)ₙ - MX₄₋ₙ (II)

wherein m represents an integer of from 1 to 4; n represents an integer of from 0 to 4, provided that m ≧ n; R represents one or, in case m is different from 1, more groups selected from hydrocarbyl residues, the same or different from one another, and optionally containing heteroatoms (e.g. O, S, N) and/or inert functional groups and the corresponding acyl groups; M represents a metal selected from Ti, Zr and Hf; X is selected from one or more of halogen (e.g. Cl and Br), CN, CNS, (cyclo)alkoxy, aryloxy and acyloxy and/or two radicals X taken together represent

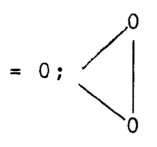

Preferably, R represents one, or in case m is different from 1, more groups selected from C₁-C₁₄(cyclo)aliphatic and C₆-C₁₄(hetero)aromatic radicals,the same or different from each other and optionally substituted and/or interrupted by groups which are inert under the reaction conditions, such as (preferably C₁₋₄-)alkyl,(preferably C₁₋₄-)alkoxy, CO, NH₂, OH and halogen (e.g. Cl and F) and from acyl residues derived from the corresponding (cyclo)aliphatic and (hetero)aromatic carboxylic acids; M represents a titanium or zirconium atom; the group or groups X represent acyloxy groups derived from carboxylic acids as defined above, C₁-C₁₄ (cyclo)alkoxy groups, C₆-C₁₄ (hetero)aryloxy groups, F, Cl, Br, CN and/or CNS, and/or two symbols X taken together may give rise to the group = 0.

Most preferably, R represents straight or branched C₁-C₅ alkyl, phenyl, naphthyl, indenyl, anisyl, cyclopentadienyl, (preferably C₁₋₆-)acyl and benzoyl; X is selected from R₁-COO-alkanoyloxy groups, wherein R₁ is C₁-C₅ alkyl or benzyl, C₆H₅-COO-benzyloxy groups, C₁-C₅ alkoxy (methoxy, ethoxy, propoxy, butoxy, etc.) groups, phenoxy groups and halogen atoms (Cl, Br); and M is Ti or Zr.

The following titanium and zirconium compounds of formula II have proven to be particularly active catalysts: titanium (tetra)ethylate, titanium(tetra)n-butylate, titanium(tetra)isopropylate, titanium diisopropyloxydiacetylacetonate, titanyl 8-hydroxyquinolinate, titanium acetylacetonate, bis(cyclopentadienyl) titanium dichloride, bis(cyclopentadienyl) titanium dibutylate, bis(cyclopentadienyl) titanium diphenate, bis(cyclopentadienyl) titanium dinaphthoate, bis(1,1-dinaphthyl-2,2'-diyl) orthotitanate, alpha-naphthyl titanium tributylate, indenyl titanium tributylate, dicyclopentadienylmethyl titanium chloride, zirconium(tetra)ethylate, zirconium acetylacetonate, di-n-butyl-di(2,6-di-tert.-butyl-p-cresyl) titanate, n-butyl-trioleyl titanate, tetra-o-cresyl titanate, titanium naphthenate, titanium stearate, titanium caproate, bis-acetate-titanium dichloride.

As mentioned above, the catalyst can also be selected from the salts and oxides of the above metals of Group IV B of the Periodic System. The oxides may also be utilized in an associated form, for example supported on Si0₂, for example such as Ti0₂/Si0₂.

Ti0₂/Si0₂, Ti0₂, its saline derivatives such as titanyl sulphate, TiCl₄, etc. have also proven to be active catalysts.

The catalyst may also be supported, for example on carriers such as Si0₂, Al₂0₃, (char)coal, etc., which are utilized according to conventional techniques.

Advantageous results may be obtained by using the Ti0₂/Si0₂ catalyst prepared according to the procedure disclosed in EP-A-347,926.

Said application discloses solid compositions consisting of silicon, titanium and oxygen, chemically combined with each other, wherein the amount of titanium, expressed as Ti0₂, ranges from 1 to 95% by weight with respect to the whole composition, and wherein the XR diffractogram of said composition (obtained by means of the Kα radiation of copper) shows, in the(2ϑ) range from 10° to 40°, a smooth-trend line.

As already mentioned, it is also possible to use Ti0₂/Si0₂ catalysts in the form of titanium silicalites. Titanium silicalites are well known crystalline products having a zeolitic structure and containing silicon, titanium and oxygen, such as those described in GB-A-2,071,071 and 2,024,790; US-A-4,410,501 and 4,480,135; EP-A-208,311 and 299,430.

The catalyst based on salts, oxides etc., as defined above, may be utilized in conventional physical form, for example as granules, or in the form of powders in a suspension etc., operating in a heterogeneous phase.

The process of the present invention is conducted in the liquid phase, which consists of the saturated primary amine substrate of formula (I), if it is in the liquid state, or, preferably,of said amine dissolved in an organic solvent such as acetonitrile, dioxane, dimethoxyethane, diglyme, C₁-C₁₀ alcohols (e.g. ethanol, isopropanol and tert.-butanol), aromatic hydrocarbons (such as toluene and the xylenes), dimethylformamide, dimethylsulphoxide, lower (C₃₋₁₂-)trialkylamines (e.g. triethylamine). It is also possible to use water.

When the oxidation is carried out in the presence of a solvent the substrate concentration preferably ranges from 10 to 40%, particularly from 10% to 15% by weight.

Usually, the process of the present invention is conducted in one step by directly charging an autoclave with the saturated primary amine, as such, if liquid, or in solutions, the catalyst and 0₂ in the gaseous state, under pressure.

The pressure employed generally ranges from 303.9 to 5065 KPa (3 to 50 atmospheres) and preferably from 1519.5 to 3545.5 KPa (15 to 35 atmospheres). Furthermore, the present process usually is conducted at a temperature higher than 50°C, usually in the range from 50° to 150°C, preferably from 90° to 130°C.

Gaseous molecular oxygen may also be utilized in the form of air or of mixtures with inert gases.

Preferably the catalyst is utilized in an amount ranging from 0.005 to 0.3 gram atoms of metal (Ti, Zr, Hf) contained in the catalyst per mole of substrate.

The reaction times generally range - depending on the operative conditions - from about 2 to about 4 hours.

The isolation of the oxime may be conducted in conventional manner.

The invention will be described in more detail in the following examples, which are given for illustrative purposes only and are not to be considered a limitation of the scope of the invention.

### Example 1

Into a 100 ml stainless steel autoclave, equipped with magnetic stirrer, heating system and ramps for charging and discharging gases, there were introduced:
2.97 g (3.43 ml) of cyclohexylamine (about 30 millimols),
12 ml of diglyme, and
0.4 g of tetramethyl orthotitanate (about 1.75 millimols).

The autoclave was closed and 0₂ was introduced up to a pressure of about 30 kg/cm² gauge. The whole mixture was then heated to 100°C and was reacted for 4 hours under intense stirring.

After cooling, the residual pressure was discharged, the autoclave was opened, the reaction mass was diluted with diethyl ether, filtration was effected to remove the catalyst, and the filtrate was analyzed by gas chromatography.

A selectivity in cyclohexanone oxime of about 50% was obtained, the cyclohexylamine conversion being 62%.

### Example 2

Example 1 was repeated, using 15 ml of diethylene glycol dimethyl ether (diglyme) and a temperature of 110°C.

A selectivity in cyclohexanone oxime equal to 44% and a cyclohexylamine conversion of 81% were obtained.

### Example 3

Example 1 was repeated, using tert.-butanol as solvent and a temperature of 95°C.

A selectivity in oxime equal to 51.8% was obtained, the cyclohexylamine conversion being 63%.

### Example 4

Example 1 was repeated, using an 0₂ pressure of 20 kg/cm² gauge instead of 30 kg/cm² gauge.

A selectivity in oxime equal to 50% was obtained, the amine conversion being 48.3%.

### Example 5

Example 1 was repeated, using 12 ml of H₂0 as solvent and a temperature of 120°C for 2 hours.

A selectivity in oxime equal to 16% was obtained, the amine conversion being 21%.

### Example 6

Example 1 was repeated, using 12 ml of dimethylformamide as solvent and a temperature of 90°C for 3 hours.

A selectivity in oxime equal to 44.3% was obtained, the amine conversion being 68.2%.

### Example 7

Example 6 was repeated, using CH₃CN as solvent.

A selectivity in oxime equal to 15% was obtained, the amine conversion being 28.8%.

### Example 8

Example 6 was repeated, using triethylamine as solvent.

A selectivity in oxime equal to 37.3% was obtained, the amine conversion being 37.5%.

### Example 9

Example 1 was repeated, using 0.415 g (1.75 millimols) of bis-acetate titanium dichloride as catalyst.

A selectivity in oxime equal to 35% was obtained, the amine conversion being 96%.

### Example 10

Example 1 was repeated, using tert.-butanol as solvent and 0.855 g (1.75 millimols) of zirconium tetra-acetylacetonate as catalyst.

A selectivity in oxime equal to 25% was obtained, the amine conversion being 92%.

### Example 11 (Preparation of the titanium silicalite catalyst)

544 g of tetraethyl orthosilicate were introduced into a flame resistant Pyrex glass flask equipped with a stirrer and maintained in an inert nitrogen atmosphere. 24 g of titanium tetraisopropylate were then added and subsequently 1,200 g of an aqueous solution of tetrapropylammonium hydroxide at 20% by weight were gradually added (by dropping). The mixture was maintained under stirring for 1 hour at room temperature, then it was allowed to stand, always at room temperature, for 1 hour. The temperature was gradually raised up to 78°C to remove ethyl alcohol, and then was raised to 98°C to remove isopropyl alcohol. The removal of the alcohols generated in the course of the reaction was carried out, under stirring, within 5 hours. After cooling, the volume of the liquid was brought to 2 litres by addition of deionized water and the resulting (homogeneous and opalescent) solution was transferred into an autoclave equipped with stirrer, where the hydrothermal synthesis was effected at 175°C for 10 days under autogenous pressure.

The reaction mixture was cooled and filtered, and the solid product was repeatedly washed to a neutral pH, whereafter it was dried at 120°C for 15 hours. The dried product finally was calcined at 420°C for 10 hours.

The calcined product was placed into a beaker and mixed with an aqueous solution prepared by adding 100 ml of hydrogen peroxide (at 30% by weight) along with 1,000 ml of diluted sulphuric acid (at 5% by weight); mixing was continued for two hours at 70°C, then the liquid was separated by decantation. This operation was repeated twice with fresh solutions, and after the last acid washing a filtration was carried out, followed by a long washing with deionized water (to neutral pH). The resulting product was dried at 120°C for 15 hours and then calcined at 550°C for 2 hours. On analysis, the content of Ti as such was equal to 0.9%.

### Example 12

Example 1 was repeated, using 7 ml of diglyme instead of 12 ml, a temperature of 120°C and 1 g of powdered titanium silicalite, prepared as in example 11, as catalyst.

A selectivity in oxime equal to 30% was obtained, the amine conversion being 36.5%.

### Example 13 (Preparation of the Ti0₂, catalyst)

To 100 g of titanium tetraisopropylate there were added, dropwise and under stirring, 100 ml of distilled water. The mass was maintained under stirring for 4 hours at room temperature.

After filtration and careful washing with water, the precipitate was dried for 16 hours at 120°C and subsequently at 200°C for 2 hours.

The resulting product had a specific surface area of 255 m²/g.

### Example 14

Example 12 was repeated, using 0.4 g of powdered Ti0₂, prepared in example 13, as catalyst.

A selectivity in oxime equal to 27.9% was obtained, the amine conversion being 35%.

### Example 15

Example 1 was repeated, using 2.55 g (about 30 millimols) of cyclopentylamine as substrate, 12 ml of tert.-butanol as solvent and a reaction time of 3 hours.

A selectivity in cyclopentanone oxime equal to 25.1% was obtained, the conversion of cyclopentylamine being 96%.

### Example 16

Example 15 was repeated, using 3.46 g (30 millimols) of heptylamine as substrate.

A selectivity in oxime equal to 22.4% was obtained, the heptylamine conversion being 50.6%.

### Example 17 (Preparation of the Ti0₂/Si0₂ catalyst according to EP-A-347,926)

50 g of an amorphous microspheroidal silica having a specific surface area of 408 m²/g and a pore volume equal to 2.10 cm³/g were calcined at 300°C for 1 hour and subsequently impregnated with 115 ml of a solution consisting of 35 ml of tetraisopropyl-orthotitanate and 80 ml of isopropyl alcohol, which solution had previously been dehydrated on a molecular sieve (Zeolite 4A). The resulting impregnated silica was allowed to stand for 4 hours at room temperature; then it was dried at 120°C for 16 hours. The resulting catalyst contained 16.4% by weight of titanium, expressed as Ti0₂.

### Example 18

Example 1 was repeated by using 12 ml of tert.-butanol as solvent, a temperature of 110°C, a reaction time of 3 hours and 1 g of Ti0₂/Si0₂, prepared in example 17 in the form of a powder, as catalyst.

A selectivity of cyclohexanone oxime equal to 48.5% was obtained; the cyclohexylamine conversion was 53%.

## Claims

1. Process for preparing (cyclo)aliphatic oximes by reacting the corresponding saturated aliphatic or cycloaliphatic primary amine, which contains a C-H bond in alpha position with respect to the amine group, with oxygen, characterized in that the reaction is conducted in the liquid phase and in the presence of a catalyst comprising at least one compound of a metal of Group IV B of the Periodic System.

2. Process according to claim 1, characterized in that the saturated (cyclo)aliphatic primary amine has the formula: 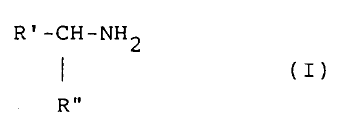 wherein R' represents a straight or branched alkyl group, R'' represents hydrogen or a straight or branched alkyl group, the same or different from R', or R' and R'', taken together, give rise to a cycloaliphatic ring, R' and R'' having a total of up to 15 carbon atoms and being optionally substituted by groups which are inert under the reaction conditions.

3. Process according to claim 1, characterized in that the starting amine is selected from cyclohexylamine, cyclopentylamine, (cyclo)heptylamine and cyclododecylamine, said amines being optionally substituted by groups which are inert under the reaction conditions.

4. Process according to any of the preceding claims, characterized in that it is conducted in the presence of a catalyst selected from the salts, oxides and metallorganic compounds of the metals of Group IV B of the Periodic System.

5. Process according to any of the preceding claims, characterized in that the catalyst is selected from compounds of Ti and Zr.

6. Process according to any of the preceding claims, characterized in that the catalyst comprises at least one metallorganic compound of formula (II):
Rₘ (0)ₙ - MX₄₋ₘ (II)
wherein m represents an integer of from 1 to 4; n represents an integer of from 0 to 4, provided that m ≧ n; R represents groups selected from hydrocarbyl radicals, the same or different from one another and optionally containing heteroatoms and/or inert functional groups, and the corresponding acyl groups; M represents a metal selected from Ti, Zr and Hf; X is selected from halogen, CN, CNS, (cyclo)alkoxy, aryloxy, and acyloxy or two radicals X taken together represent

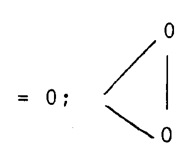

7. Process according to claim 6, characterized in that the catalyst comprises at least one compound of formula (II) in which R represents C₁-C₁₄ (cyclo)aliphatic and/or C₆-C₁₄ (hetero)aromatic residues, the same or different from one another and optionally substituted and/or interrupted by groups which are inert under the reaction conditions, such as alkyl groups, lower alkoxy groups, CO, NH₂, OH and halogen, or R represents acyl residues derived from the corresponding (cyclo)aliphatic and/or (hetero)aromatic carboxylic acids; M represents Ti or Zr; the group or groups X represent acyloxy groups derived from said carboxylic acids, C₁-C₁₄ (cyclo)alkoxy groups, C₆-C₁₄ (hetero)aryloxy groups, F, Cl, Br, CN and/or CNS, or two symbols X, taken together, give rise to the group = 0.

8. Process according to any of claims 6 and 7, characterized in that the catalyst comprises at least one compound of formula (II) in which R represents straight or branched C₁-C₅ alkyl, phenyl, naphthyl, indenyl, anisyl, cyclopentadienyl, acyl and benzoyl groups, X is selected from R₁-COO- in which R₁ is C₁-C₅ alkyl or benzyl, C₆H₅-COO-, C₁-C₅ alkoxy, phenoxy, Cl and/or Br; and M is Ti or Zr.

9. Process according to any of the preceding claims, characterized in that the catalyst comprises at least one of titanium ethylate, titanium n-butylate, titanium isopropylate, titanium diisopropyloxydiacetyl acetonate, titanyl 8-hydroxyquinolinate, titanium acetyl acetonate, bis(cyclopentadienyl) titanium dichloride, bis(cyclopentadienyl) titanium dibutylate, bis(cyclopentadienyl) titanium diphenate, bis(cyclopentadienyl) titanium dinaphthoate, bis(1,1-dinaphthyl-2,2'-diyl)-orthotitanate, alpha-naphthyl titanium tributylate, indenyl titanium tributylate, dicyclopentadienyl methyl titanium chloride, zirconium ethylate, zirconium acetylacetonate, di-n-butyl-di-(2,6-di-tert.-butyl-p-cresyl) titanate, n-butyl-trioleyl titanate, tetra-o-cresyl titanate, titanium naphthenate, titanium stearate, titanium caproate and bis-acetate-titanium dichloride.

10. Process according to any of the preceding claims, characterized in that the catalyst comprises at least one of Ti0₂, titanyl sulphate, titanium tetrachloride and titanium silicalites.

11. Process according to any of the preceding claims, characterized in that the catalyst is supported on and/or is associated with Si0₂, Al₂0₃ and charcoal.

12. Process according to any of the preceding claims, characterized in that the catalyst comprises a composition of Ti0₂ and Si0₂ in chemically combined form, wherein the amount of Ti0₂ ranges from 1 to 95% by weight, based on said composition, and the XR diffractogram of said composition (obtained by means of the Kα radiation of copper) shows, in the (2ϑ) range from 10° to 40°, a smooth-trend line.

13. Process according to any of the preceding claims, characterized in that the catalyst is utilized in an amount of from 0.005 to 0.3 gram atoms of metal contained in said catalyst per mole of substrate.

14. Process according to any of the preceding claims, characterized in that the liquid phase consists of excess saturated (cyclo)aliphatic primary amine to be oxidized.

15. Process according to any of claims 1 to 13, characterized in that the amine is utilized in solution in an organic solvent and/or water, said organic solvent being preferably selected from acetonitrile, dioxane, dimethoxyethane, diglyme, C₁-C₁₀ alcohols, aromatic hydrocarbons, dimethylformamide, dimethylsulphoxide, triethylamine and mixtures thereof.

16. Process according to claim 15, characterized in that the concentration of the substrate in the solvent ranges from 10% to 40% by weight.

17. Process according to any of the preceding claims, characterized in that oxygen is introduced in the form of air or in admixture with inert gases.

18. Process according to any of the preceding claims, characterized in that the pressure ranges from 303.9 to 5065 KPa (3 to 50 atmospheres) and preferably from 1519.5 to 3545.5 KPa (15 to 35 atmospheres).

19. Process according to any of the preceding claims, characterized in that the temperature ranges from 50°C to 150°C, preferably from 90°C to 130°C.

## Patentansprüche

1. Verfahren zur Herstellung (cyclo)aliphatischer Oxime durch Umsetzung des entsprechenden gesättigten aliphatischen oder cycloaliphatischen primären Amins, das eine C-H-Bindung in α-Stellung zur Aminogruppe enthält, mit Sauerstoff, dadurch gekennzeichnet, daß die Umsetzung in flüssiger Phase und in Anwesenheit eines Katalysators erfolgt, der wenigstens eine Verbindung eines Metalls der Gruppe IV B des periodischen Systems umfaßt.

2. Verfahren nach Anspruch 1, dadurch gekennzeichnet, daß das gesättigte (cyclo)aliphatische primäre Amin die folgende Formel hat: 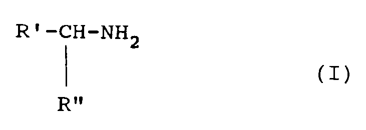 worin R' eine gerade oder verzweigte Alkylgruppe bedeutet,
R'' Wasserstoff oder eine gerade oder verzweigte Alkylgruppe bedeutet, die gleich oder verschieden von R' ist, oder R' und R'' zusammen einen cycloaliphatischen Ring bilden, R' und R'' insgesamt bis zu 15 Kohlenstoffatome enthalten und gegebenenfalls durch Gruppen substituiert sind, die unter den Reaktionsbedingungen inert sind.

3. Verfahren nach Anspruch 1, dadurch gekennzeichnet, daß das Ausgangsamin ausgewählt wird aus Cyclohexylamin, Cyclopentylamin, (Cyclo)heptylamin und Cyclododecylamin, wobei die genannten Amine gegebenenfalls durch Gruppen substituiert sind, die unter den Reaktionsbedingungen inert sind.

4. Verfahren nach einem der vorhergehenden Ansprüche, dadurch gekennzeichnet, daß dieses in Anwesenheit eines Katalysators, ausgewählt aus Salzen, Oxiden und metallorganischen Verbindungen der Metalle der Gruppe IV B des periodischen Systems, durchgeführt wird.

5. Verfahren nach einem der vorhergehenden Ansprüche, dadurch gekennzeichnet, daß der Katalysator aus Verbindungen von Ti und Zr ausgewählt wird.

6. Verfahren nach einem der vorhergehenden Ansprüche, dadurch gekennzeichnet, daß der Katalysator wenigstens eine metallorganische Verbindung der Formel (II):
Rₘ (O)ₙ - MX₄₋ₘ
umfaßt, worin m eine ganze Zahl von 1 bis 4 ist; n eine ganze Zahl von 0 bis 4 ist unter der Voraussetzung, daß m ≧ n ist; R eine Gruppe bedeutet, die ausgewählt wird aus Hydrocarbyl-Radikalen, die gleich oder verschieden sein können und gegebenenfalls Heteroatome und/oder inerte funktionelle Gruppen enthalten, und den entsprechenden Acylgruppen; M ein Metall bedeutet, ausgewählt aus Ti, Zr und Hf; X ausgewählt wird aus Halogen, CN, CNS, (Cyclo)alkoxy, Aryloxy und Acyloxy oder zwei Radikale X zusammen 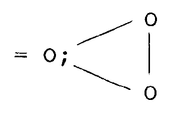 bedeuten.

7. Verfahren nach Anspruch 6, dadurch gekennzeichnet, daß der Katalysator wenigstens eine Verbindung der Formel (II) umfaßt, worin R für C₁-C₁₄-(cyclo)aliphatische und/oder C₆-C₁₄-(hetero)aromatische Reste steht, die gleich oder verschieden und gegebenenfalls substituiert sein können und/oder von Gruppen unterbrochen sein können, die unter den Reaktionsbedingungen inert sind, wie Alkylgruppen, niedere Alkoxygruppen, CO, NH₂, OH und Halogen, oder R Acylreste darstellt, die von den entsprechenden (cyclo)aliphatischen und/oder (hetero)aromatischen Carbonsäuren abgeleitet sind; M = Ti oder Zr bedeutet; die Gruppe oder die Gruppen X Acyloxygruppen, abgeleitet von den genannten Carbonsäuren, C₁-C₁₄(Cyclo)alkoxygruppen, C₆-C₁₄ (Hetero)aryloxygruppen, F, Cl, Br, CN und/oder CNS bedeuten, oder zwei Symbole X zusammen eine Gruppe = O bilden.

8. Verfahren nach einem der Ansprüche 6 und 7, dadurch gekennzeichnet, daß der Katalysator wenigstens eine Verbindung der Formel (II) umfaßt, worin R gerade oder verzweigte C₁-C ₅-Alkyl-, Phenyl-, Naphthyl-, Indenyl-, Anisyl-, Cyclopentadienyl-, Acyl- und Benzoylgruppen darstellt, X ausgewählt wird aus R₁-COO-, worin R₁ C₁-C₅-Alkyl oder Benzyl bedeutet, C₆H₅-COO-, C₁-C₅-Alkoxy, Phenoxy, Cl und/oder Br, und M = Ti oder Zr ist.

9. Verfahren nach einem der vorhergehenden Ansprüche, dadurch gekennzeichnet, daß der Katalysator wenigstens eine der Verbindungen Titanethylat, Titan-n-butylat, Titan-isopropylat,Titandiisopropyloxydiacetyl-acetonat, Titanyl-8-hydroxychinolinat, Titanacetyl-acetonat, Bis-(cyclopentadienyl)-titandichlorid, Bis-(cyclopentadienyl)-titandibutylat, Bis(cyclopentadienyl)titandiphenolat, Bis(cyclopentadienyl)titandinaphthoat, Bis(1,1-dinaphthyl-2,2'-diyl)-orthotitanat, α-Naphthyltitantributylat, Indenyltitantributylat, Dicyclopentadienylmethyltitanchlorid, Zirkonethylat, Zirkonacetylacetonat, Di-n-butyl-di(2,6-di-tert.butyl-p-cresyl)titanat, n-Butyl-trioleyltitanat, Tetra-o-cresyltitanat, Titannaphthenat, Titanstearat, Titancaproat und Bis-acetattitandichlorid umfaßt.

10. Verfahren nach einem der vorhergehenden Ansprüche, dadurch gekennzeichnet, daß der Katalysator wenigstens eine der Verbindungen TiO₂, Titanylsulfat, Titantetrachlorid und Titansilikalit umfaßt.

11. Verfahren nach einem der vorhergehenden Ansprüche, dadurch gekennzeichnet, daß der Katalysator auf einem Träger aus SiO₂, Al₂O₃ und Holzkohle und/oder mit diesen Materialien kombiniert ist.

12. Verfahren nach einem der vorhergehenden Ansprüche, dadurch gekennzeichnet, daß der Katalyssator eine Zusammensetzung aus TiO₂ und SiO₂ in chemisch kombinierter Form umfaßt, worin die Menge an TiO₂ von 1 bis 95 Gew.-%, bezogen auf die genannte Zusammensetzung, beträgt und das XR-Diffraktogramm der genannten Zusammensetzung (erhalten mittels der Kα-Bestrahlung von Kupfer) in dem (2ϑ)-Bereich von 10° bis 40° eine annähernd gleichförmige Linie zeigt.

13. Verfahren nach einem der vorhergehenden Ansprüche, dadurch gekennzeichnet, daß der Katalysator in einer Menge von 0,005 bis 0,3 Grammatomen Metall verwendet wird, das in dem Katalysator pro Mol des Substrats enthalten ist.

14. Verfahren nach einem der vorhergehenden Ansprüche, dadurch gekennzeichnet, daß die flüssige Phase aus überschüssigem, gesättigten (cyclo)aliphatischen primären zu oxidierenden Amin besteht.

15. Verfahren nach einem der Ansprüche 1 bis 13, dadurch gekennzeichnet, daß das Amin in Lösung in einem organischen Lösungsmittel und/oder Wasser verwendet wird, wobei das genannte Lösungsmittel vorzugsweise ausgewählt wird aus Acetonitril, Dioxan, Dimethoxyethan, Diglyme, C₁-C₁₀-Alkoholen, aromatischen Kohlenwasserstoffen, Dimethylformamid, Dimethylsulfoxid, Triethylamin und Mischungen von diesen.

16. Verfahren nach Anspruch 15, dadurch gekennzeichnet, daß die Konzentration des Substrats in dem Lösungsmittel von 10 Gew.-% bis 40 Gew.-% beträgt.

17. Verfahren nach einem der vorhergehenden Ansprüche, dadurch gekennzeichnet, daß der Sauerstoff in Form von Luft oder in Mischung mit inerten Gasen eingeführt wird.

18. Verfahren nach einem der vorhergehenden Ansprüche, dadurch gekennzeichnet, daß der Druck von 303,9 bis 5065 KPa (3 bis 50 at) und vorzugsweise von 1519,5 bis 3545,5 KPa (15 bis 35 at) beträgt.

19. Verfahren nach einem der vorhergehenden Ansprüche, dadurch gekennzeichnet, daß die Temperatur von 50° C bis 150° C, vorzugsweise von 90° C bis 130° C, beträgt.

## Revendications

1. Procédé de préparation d'oximes (cyclo)aliphatiques par réaction de l'amine primaire aliphatique ou cycloaliphatique saturée correspondante, qui contient une liaison C-H en position alpha par rapport au groupe amine, avec de l'oxygène, caractérisé en ce que la réaction est mise en oeuvre en phase liquide et en présence d'un catalyseur contenant au moins un dérivé d'un métal du groupe IVB du Tableau Périodique.

2. Un procédé selon la revendication 1, caractérisé en ce que l'amine primaire (cyclo)aliphatique saturée est représentée par la formule: 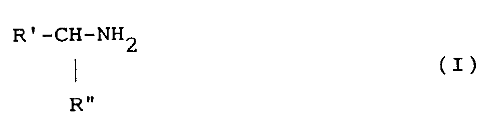 dans laquelle:
R' représente un radical alkyle linéaire ou ramifié;
R'' représente un atome d'hydrogène ou un radical alkyle linéaire ou ramifié, qui est identique ou différent de R'; ou
R' et R'' forment ensemble un cycle cycloaliphatique, R' et R'' comportant au total jusqu'à 15 atomes de carbone et étant éventuellement substitués par des groupes qui sont inertes dans les conditions de réaction.

3. Un procédé selon la revendication 1, caractérisé en ce que l'amine de départ est sélectionnée parmi: cyclohexylamine, cyclopentylamine, (cyclo)heptylamine et cyclododécylamine, ces amines étant éventuellement substituées par des groupes qui sont inertes dans les conditions de réaction.

4. Un procédé selon l'une quelconque des revendications précédentes, caractérisé en ce qu'il est mis en oeuvre en présence d'un catalyseur sélectionné parmi les sels, oxydes et composés organométalliques des métaux du groupe IVB du Tableau Périodique.

5. Un procédé selon l'une quelconque des revendications précédentes, caractérisé en ce que le catalyseur est sélectionné parmi les composés de Ti et de Zr.

6. Un procédé selon l'une quelconque des revendications précédentes, caractérisé en ce que le catalyseur comprend au moins un composé organométallique de formule (II):
Rₘ (O)ₙ - MX₄₋ₙ (II)
dans laquelle:
m représente un nombre entier de 1 à 4;
n représente un nombre entier de 0 à 4; à condition que m ≧ n;
R représente des groupes sélectionnés parmi les radicaux hydrocarbyles, identiques ou différents les uns des autres et qui contiennent éventuellement des hétéroatomes et/ou des groupes fonctionnels inertes et parmi les radicaux acyles correspondants:
M représente un métal sélectionné parmi Ti, Zr et Hf;
X est un ou plusieurs des radicaux du groupe formé par les atomes d'halogène, CN, CNS, (cyclo)alcoxy, aryloxy et acyloxy ou deux radicaux X forment ensemble:

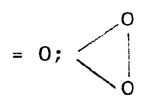

7. Un procédé selon la revendication 6, caractérisé en ce que le catalyseur comprend au moins un composé représenté par la formule (II), dans laquelle:
R représente un résidu (hétéro)aromatique en C₆ à C₁₄ et/ou un résidu (cyclo)aliphatique en C₁ à C_{14,} ces résidus pouvant être identiques ou différents les uns des autres et éventuellement substitués et/ou interrompus par des groupes qui sont inertes dans les conditions de réaction, tels que des radicaux alkyles, alcoxy inférieurs, CO, NH₂, OH ou un atome d'halogène; ou
R représente des résidus acyles dérivant des acides carboxyliques (cyclo)aliphatiques et/ou (hétéro )aromatiques correspondants;
M représente Ti ou Zr;
le groupe ou les groupes X représentent des radicaux acyloxy
dérivant desdits acides carboxyliques, des radicaux (cyclo)alcoxy en C₁ à C_{14,} des radicaux (hétéro)aryloxy en C₆ à C₁₄, F, Cl, Br, CN et/ou CNS; ou deux groupes X forment ensemble un groupe = O.

8. Un procédé selon l'une des revendications 6 et 7, caractérisé en ce que le catalyseur comprend au moins un composé représenté par la formule (II), dans laquelle:
R représente un radical benzoyle, acyle, cyclopentadiényle, anisyle, indényle, naphtyle, phényle, acyle en C₁ à C₅, linéaire ou ramifié;
X est sélectionné parmi le groupe R₁-COO-, dans lequel: R₁ représente un radical benzyle ou alkyle en C₁ à C₅, C₆H₅-COO-, un radical alcoxy en C₁ à C₅, un radical phénoxy, Cl et/ou Br; et
M représente Ti ou Zr.

9. Un procédé selon l'une quelconque des revendications précédentes, caractérisé en ce que le catalyseur comprend au moins l'un des composés suivants: éthylate de titane, n-butylate de titane, isopropylate de titane, diisopropyloxydiacétylacétonate de titane, 8-hydroxyquinolinate de titanyle, acétylacétonate de titane, dichlorure de bis(cyclopentadiényl)-titane, dibutylate de bis(cyclopentadiényl)titane, diphénate de bis(cyclopentadiényl)titane, dinaphtonate de bis(cyclopentadiényl)titane, bis(1,1-dinaphtyl-2,2'-diyl)orthotitanate, tributylate d'alphanaphtyltitane, tributylate d'indényltitane, chlorure de dicyclopentadiénylméthyltitane, éthylate de zirconium, acétylacétonate de zirconium, di-n-butyl-(2,6-di-ter.-butyl-p-crésyl)titanate, n-butyl-trioléyl-titanate, tétra-o-crésyl-titanate, naphténate de titane, stéarate de titane, caproate de titane, dichlorure de bis-acétate-titane.

10. Procédé selon l'une quelconque des revendications précédentes, caractérisé en ce que le catalyseur comprend au moins l'un des composés suivants: TiO₂, sulfate de titanyle, tétrachlorure de titane et silicalite de titane.

11. Procédé selon l'une quelconque des revendications précédentes, caractérisé en ce que le catalyseur est fixé sur un support formé de et/ou associé, à SiO₂, Al₂O₃ et charbon de bois.

12. Procédé selon l'une quelconque des revendications précédentes, caractérisé en ce que le catalyseur comprend une composition de TiO₂ et SiO₂, sous une forme combinée chimiquement, dans laquelle la quantité de TiO₂ est comprise dans un intervalle allant de 1 à 95% en poids, par rapport à ladite composition et le spectre de diffraction au rayons X de ladite composition (obtenu par utilisation de la radiation Kα du cuivre) présente dans l'intervalle (2ν) allant de 10 à 40°, une ligne à faible pente.

13. Procédé selon l'une quelconque des revendications précédentes, caractérisé en ce que le catalyseur est utilisé en une quantité de 0,005 à 0,3 g d'atome de métal contenu dans ledit catalyseur par mole de substrat.

14. Procédé selon l'une quelconque des revendications précédentes, caractérisé en ce que la phase solide est constituée de l'amine primaire (cyclo)aliphatique saturée en excès devant être oxydée.

15. Procédé selon l'une quelconque des revendications 1 à 13, caractérisé en ce que l'amine est utilisée en solution dans un solvant organique et/ou dans l'eau, ledit solvant organique étant de préférence sélectionné parmi: acétonitrile, dioxane, diméthoxyéthane, diglyme, alcools en C₁ à C₁₀, hydrocarbure aromatique, diméthylformamide, diméthylsulfoxyde, triéthylamine et leurs mélanges.

16. Procédé selon la revendication 15, caractérisé en ce que la concentration en substrat dans le solvant est comprise dans un intervalle allant de 10% à 40%.

17. Procédé selon l'une quelconque des revendications précédentes, caractérisé en ce que l'oxygène est introduit sous la forme d'air ou sous la forme de mélange avec des gaz inertes.

18. Procédé selon l'une quelconque des revendications précédentes, caractérisé en ce que la pression est comprise dans un intervalle allant de 303,9 à 5065 kPa (3 à 50 atmosphères) et de préférence dans un intervalle allant de 1519,5 à 3545,5 kPa (15 à 35 atmosphères).

19. Procédé selon l'une quelconque des revendications précédentes, caractérisé en ce que la température est comprise dans un intervalle allant de 50°C à 150°C, et de préférence dans un intervalle allant de 90°C à 130°C.
